# EUROPEAN PATENT SPECIFICATION

(11) **EP 3 082 578 B1**
(45) Date of publication and mention of the grant of the patent: **10.07.2019**
(21) Application number: 14833193.7
(22) Date of filing: 08.12.2014
(51) Int. Cl.: A61B 5/00, A61B 5/01, G01K 1/16

(54) **VITAL SIGN MONITORING AND CONTROL**
ÜBERWACHUNG VON VITALZEICHEN UND STEUERUNG
SURVEILLANCE ET RÉGULATION DES SIGNES VITAUX

(30) Priority: 18.12.2013 US 201361917634 P
(43) Date of publication of application: 26.10.2016
(73) Proprietor: Koninklijke Philips N.V., 5656 AE Eindhoven (NL)
(72) Inventor: MEFTAH, Mohammed, NL-5656 AE Eindhoven (NL); BONGERS, Edwin Gerardus Johannus Maria, NL-5656 AE Eindhoven (NL)
(74) Representative: Steffen, Thomas
(86) International application number: PCT/IB2014/066688
(87) International publication number: WO 2015/092607

(56) References cited:
- EP-A1- 2 406 604
- EP-A1- 2 441 385
- WO-A1-2011/007294
- DE-A1-102005 048 496

## Description

### BACKGROUND

### 1. Field

The present disclosure pertains to a system and method for non-invasive determination of one or more vital sign states of a subject, and, in particular, determining a reliability index for the one or more determined vital sign states of a subject.

### 2. Description of the Related Art

Measuring vital signs of a subject is known to be medically relevant and important in the care of a subject. For example, measuring temperatures of subjects in hospital is commonly practiced. Reducing heat loss is particularly important for preterm neonates. Specifically, the core body temperature and the peripheral temperature are important measures for diagnostic purposes, including, but not limited to, the evaluation of thermoregulation, circulatory problems, perfusion, thermoregulation issues, heat/cold stress and infections.

### SUMMARY

Accordingly, one or more embodiments provide a measuring system for non-invasive determination of one or more vital signs of a subject. The system comprises a body of engagement configured to engage with and/or support a subject, multiple coupling sensors, multiple vital sign sensors, and one or more processors configured to execute computer program modules. The coupling sensors, in some embodiments, generate coupling signals conveying electrical, thermal, and/or magnetic coupling information with the subject. The coupling sensors are generally carried by the body of engagement. The vital sign sensors generate output signals conveying vital sign information or a vital sign profile of the subject. The vital sign sensors are carried by the body of engagement. The computer program modules comprise a coupling module, a vital sign state determination module, and a quality control module. The coupling module is configured to determine coupling levels for individual ones of the vital sign sensors based on the coupling signals generated by the coupling sensors. The vital sign state determination module is configured to determine multiple vital sign states of the subject based on the output signals and, optionally, the determined coupling levels. The quality control module is configured to determine a coupling reliability index based on the coupling levels for the individual ones of the vital sign sensors.

It is yet another aspect of one or more embodiments to provide a method of non-invasive determination of one or more vital signs of a subject. The method comprises engaging a subject with a body of engagement; generating coupling signals conveying electrical, thermal, and/or magnetic coupling information with the subject at or near a point of engagement between the subject and the body of engagement; generating output signals conveying vital sign information of the subject; determining coupling levels for individual ones of the vital sign sensors based on the coupling signals; determining multiple vital sign states of the subject based on the output signals and, optionally, the determined coupling levels; and, determining a coupling reliability index based on the coupling levels for the individual ones of the vital sign sensors..

It is yet another aspect of one or more embodiments to provide a system configured to provide non-invasive determination of one or more vital sign states of a subject. The system comprises means for engaging a subject with a body; means for generating coupling signals conveying electrical, thermal, and/or magnetic coupling information with the subject at or near a point of engagement between the subject and the means for engaging; means for generating output signals conveying vital sign information of the subject between the subject and the means for engaging; means for determining coupling levels for the means for generating output signals conveying vital sign information of the subject based on the determined coupling levels; means for determining multiple vital sign states of the subject based on the output signals and, optionally, the determined coupling levels; and, means for determining a coupling reliability index based on the coupling levels for the means for generating output signals conveying vital sign information of the subject..

These and other aspects, features, and characteristics of the present disclosure, as well as the methods of operation and functions of the related elements of structure and the combination of parts and economies of manufacture, will become more apparent upon consideration of the following description and the appended claims with reference to the accompanying drawings, all of which form a part of this specification, wherein like reference numerals designate corresponding parts in the various figures. It is to be expressly understood, however, that the drawings are for the purpose of illustration and description only and are not intended as a definition of any limits.

The invention is defined by the appended claims.

### BRIEF DESCRIPTION OF THE DRAWINGS

FIGs. 1A-1B-1C schematically illustrate a system for non-invasive determination of one or more vital sign states of a subject, in accordance with one or more embodiments;
FIG. 2 schematically illustrates a measuring system in accordance with one or more embodiments;
FIGs. 3A-3B illustrate a system for non-invasive determination of one or more vital sign states of a subject, in accordance with one or more embodiments;
FIG. 4 illustrates a user interface for displaying one or more vital sign states of a subject, in accordance with one or more embodiments;
FIG. 5 illustrates a graph of multiple vital signs measured over time in accordance with one or more embodiments;
FIGs. 6A-4B illustrate vital sign profile maps in accordance with one or more embodiments;
FIGs. 7A-7B-7C illustrate measuring systems for non-invasive determination of one or more vital sign states of a subject, in accordance with one or more embodiments; and,
FIG. 8 illustrates a method for non-invasive determination of one or more vital sign states of a subject, in accordance with one or more embodiments.

### DETAILED DESCRIPTION OF EXEMPLARY EMBODIMENTS

As used herein, the singular form of "a", "an", and "the" include plural references unless the context clearly dictates otherwise. As used herein, the statement that two or more parts or components are "coupled" shall mean that the parts are joined or operate together either directly or indirectly, *i.e*., through one or more intermediate parts or components, so long as a link occurs. As used herein, "directly coupled" means that two elements are directly in contact with each other. As used herein, "fixedly coupled" or "fixed" means that two components are coupled so as to move as one while maintaining a constant orientation relative to each other.

As used herein, the word "unitary" means a component is created as a single piece or unit. That is, a component that includes pieces that are created separately and then coupled together as a unit is not a "unitary" component or body. As employed herein, the statement that two or more parts or components "engage" one another shall mean that the parts exert a force against one another either directly or through one or more intermediate parts or components. As employed herein, the term "number" shall mean one or an integer greater than one (*i.e*., a plurality).

Directional phrases used herein, such as, for example and without limitation, top, bottom, left, right, upper, lower, front, back, and derivatives thereof, relate to the orientation of the elements shown in the drawings and are not limiting upon the claims unless expressly recited therein.

The present application is directed toward the non-invasive measurement of vital sign states of a subject, and in particular, the determination of a reliability index providing an indication of the reliability of the measured vital sign state. Vital signs are measures of various physiological statistics that are obtained to assess body functions of a subject. Vital sign states of a subject may include body temperature, pulse (or heart) rate, blood pressure, respiratory rate, and/or physiological statistical measurements. Measures of vital sign states of a subject may also include pupil dilation, skin condition, urinary continence, mobility, end-tidal CO₂ levels, lung volume, breathing flow speed, menstrual cycle, glucose levels, and/or other measurements of the body.

The following description of the figures relate to the specific vital sign state of body temperature of a subject, and in particular that of a neonatal subject. One of ordinary skill in the art will appreciate and understand that the proceeding description would relate to any vital sign state measurement, and sensors related to the measurements of any vital sign state of a subject. For example, instead of temperature sensors conveying temperature information of the subject, electrocardiogram electrodes may be used that convey heart rate information of the subject. The following example of the system and method disclosed in this application is, therefore, not intended to be limiting.

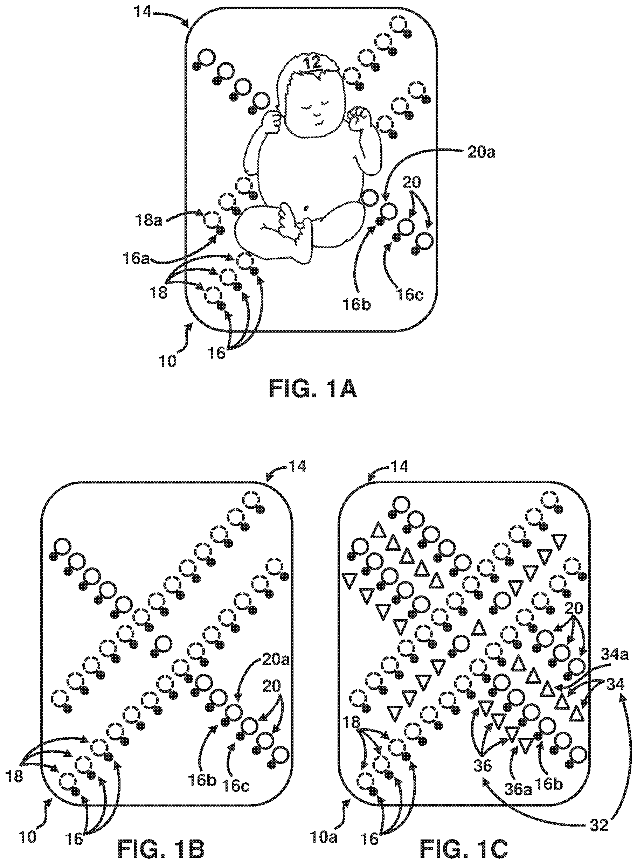

FIG. 1A illustrates (a top-view of) measuring system 10 for non-invasive determination of one or more vital sign states of subject 12. Measuring system 10 may interchangeably be referred to as system 10. Measuring system 10 may include one or more of a body of engagement 14, multiple coupling sensors 16, multiple vital sign sensors 18, and/or other components (including components illustrated in other figures as being included in measuring system 10). In this specific example of measuring system 10 for non-invasive determination of one or more temperatures of subject 12, measuring system 10 may include one or more of a body of engagement 14, multiple coupling sensors 16, multiple temperature sensors 18, one or more zero-heat-flux temperature sensors 20, and/or other components (including components illustrated in other figures as being included in measuring system 10). Body of engagement 14 may interchangeably be referred to as "structure of engagement," "structure," "support-structure of engagement," or "support-structure."

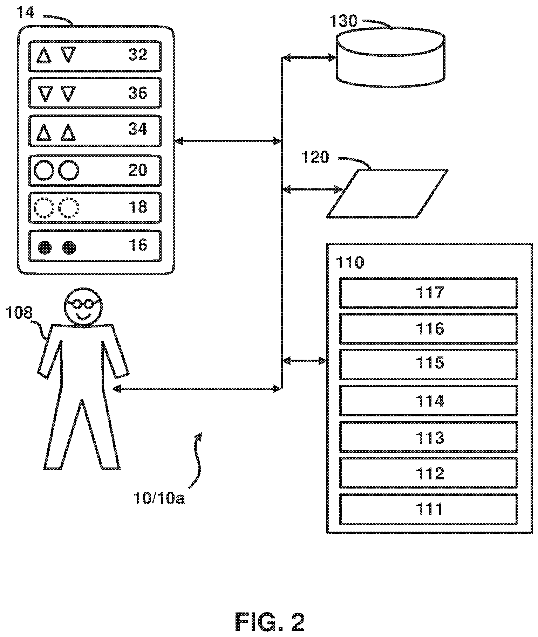

By way of non-limiting example, FIG. 2 schematically illustrates measuring system 10, which may further include one or more vital sign state adjustment elements 32 (for example, in the case of thermal adjustment elements, vital sign state adjustment elements may include one or more heating elements 34 and/or one or more cooling elements 36), one or more processors 110, an electronic storage 130, a user interface 120, and/or other components and/or computer program modules. The computer program modules may include one or more of a coupling module 111, a vital sign state determination module 112, a quality control module 113, a tracking module 114, a map module 115, a target module 116, a control module 117, and/or other modules. Also illustrated in FIG. 2 is a user 108 of measurement system 10 such as, by way of non-limiting example, a care-giver, a therapy-decision-maker, and/or a medical professional.

Non-invasive determination of one or more vital sign states of a subject, in particular neonates and/or infants, may contribute to improved medical care and/or maintenance of recommended vital sign state parameters. For example, non-invasive determination of one or more temperatures of a neonatal subject and/or infant, may contribute to thermal protection and/or maintenance of recommended temperatures. Measuring temperatures of a subject may be important in many clinical situations, including but not limited to neonates in a neonatal intensive care unit (NICU). The multiple temperatures may include peripheral temperatures at various locations, core temperatures at or near different parts of the body, and/or other temperatures. For example, peripheral temperatures may include skin temperatures of hands, feet, and/or other body parts. For example, core temperatures may include (estimated, determined, measured, and/or otherwise approximated) temperatures of various organs and/or body parts, including but not limited to the brain, the heart, the abdomen, the chest, and/or other organs and/or body parts. As used herein, the term "non-invasive" may refer to the absence of adhesives to keep sensors in place and/or the absence of physical equipment penetrating or adhering to the skin or being inserted in any manner into the subject. Adhesive (vital sign state) sensors may damage the skin and cause stress and/or pain when used. Information regarding on or more vital sign states of a subject (as well as information regarding changes over time in one or more such vital sign states) may be medically and/or diagnostically relevant. For example, issues regarding thermoregulation, circulatory function, perfusion, infections, oxygen saturation, and/or other conditions of a subject may be diagnosed, monitored, treated, and/or otherwise benefit by virtue of having more and/or more accurate information regarding one or more temperatures, and/or other vital sign state information of the subject. Medical conditions and/or issues mentioned in this disclosure are intended to be exemplary and without limitation.

Referring to FIG. 1A, body of engagement 14 is configured to engage with a subject 12, *e.g.* a neonate and/or infant. In some embodiments, body of engagement 14 may be implemented as a (subject) support structure configured to support subject 12 thereon. A subject support structure may be a mattress, a bed, a pad, a blanket, a wrap, a pillow, an incubator, and/or other structure suitable to engage and/or support a subject 12, *e.g.* a neonate and/or infant. In some embodiments, body of engagement 14 may be an article of clothing configured to be worn by and/or wrapped around subject 12. Body of engagement 14 may be configured to carry one or more sensors, e.g. one or more temperature sensors 16. As depicted in FIG. 1A, body of engagement 14 may be wrapped around subject 12 such that multiple coupling sensors 16 and multiple temperature sensors 18 engage, touch, and/or (electrically and/or thermally) couple with subject 12.

As used herein, a generic reference to a temperature sensor or a reference to multiple temperature sensors may use the term "temperature sensor(s) 18," or variations thereof using the reference numeral "18," whereas a specific individual temperature sensor may be referred to by appending a character to that reference numeral, *e.g.* "temperature sensor 18a", depicted in FIG. 1A. Likewise, FIG. 1A depicts multiple coupling sensors 16 as well as specific coupling sensors referred to as coupling sensor 16a, coupling sensor 16b, and coupling sensor 16c, and multiple zero-heat-flux temperature sensors 20 as well as a specific zero-heat-flux temperature sensor 20a. Other temperature sensors, coupling sensors and zero-heat-flux temperature sensors are depicted in FIG. 1A, but not individually labeled with a reference number. As used in any of the figures, similar types of sensors may be depicted by similar schematic symbols. For example, temperature sensor(s) 18 are depicted using similar symbols in FIGs. 1A-1B-1C and FIG. 2. The disclosure is not limited to the number or position of any sensors depicted in any of the figures. As used herein, the term "measure" refers to any combination of measuring, estimating, and/or approximating based on output generated by one or more sensors. As used herein, the term "measurement" refers to any combination of one or more measurements, estimations, and/or approximations based on output generated by one or more sensors.

Temperature sensor(s) 18 may be configured to generate output signals conveying temperatures of a subject and/or output signals conveying information related in a predictable manner (*e.g*. through a mathematical relationship) to one or more temperatures of a subject. In some embodiments, temperature sensor(s) 18 may include one or more zero-heat-flux temperature sensors 20. Temperature sensor(s) may be supported and/or carried by body of engagement 14. Zero-heat-flux temperature sensor(s) 20 may be configured to create thermal insulation between two objects (e.g. body of engagement 14 and subject 12). Zero-heat-flux temperature sensor(s) 20 operate according to the thermal principle known as the zero-heat flux principle, which may be described, *e.g*., in one or more related applications incorporated by reference into the present application. In some embodiments, temperature sensor(s) 18 may be used to determine one or more peripheral temperatures of subject 12. In some embodiments, zero-heat-flux temperature sensor(s) 20 may be used to determine one or more core temperatures of subject 12. In some embodiments, one or more temperature sensors 18 may be configured to determine an ambient temperature around and/or near subject 12.

Coupling sensors 16 may be configured to generate signals (interchangeably referred to herein as output signals or coupling signals) conveying coupling information between two objects (*e.g*. the coupling sensor itself and subject 12). Coupling sensors 16 may be configured to generate signals conveying electrical, thermal, magnetic, pressure, and/or other coupling information. Coupling sensor(s) 16 may be supported and/or carried by body of engagement 14. In some embodiments, coupling sensor(s) may include one or more magnetic field sensors, one or more pressure sensors and/or one or more capacitive sensors. Signals and/or information conveyed by coupling sensor(s) 16 may be referred to as coupling information. One or more coupling sensors 16 may be associated with one or more temperature sensors, including but not limited using a 1-to-1 association (*e.g.* for co-located sensor pairs of a temperature sensor and a coupling sensor). By way of non-limiting example, referring to FIG. 1A, coupling sensors 16a, 16b, and 16c may be associated with different (zero-heat-flux) temperature sensors. In some embodiments, coupling information may be conveyed by the intensity, strength, magnitude, and/or level of the signal generated by coupling sensor(s) 16. For example, in some embodiments, an individual coupling sensor 16 may emit a signal (*e.g.* an electromagnetic signal) having known characteristics (including but not limited to a known frequency, shape, magnitude, and/or other characteristic of an electromagnetic signal). The coupling information for the individual coupling sensor 16 may be based on how well the emitted signal is received. In case of good and/or strong coupling between the coupling sensor and subject 12, the received signal may have a greater magnitude than compared to a poor and/or weak coupling between the coupling sensor and subject 12.

In some embodiments, an individual coupling sensor may be associated with multiple temperature sensors. In some embodiments, multiple coupling sensors may be associated with an individual temperature sensor. In some embodiments, association between one or more coupling sensors 16 and one or more temperature sensors 18 may be based on proximity (including but not limited to a weighted association of the information from a temperature sensor based on coupling information from the nearest multiple coupling sensors). In some embodiments, an individual temperature sensor and an individual coupling sensor may be integrated, embedded, and/or otherwise combined into a single unit, component, and/or device capable of the joint features and functionality attributed herein to an individual temperature sensor and an individual coupling sensor.

By way of non-limiting example, coupling sensor 16 depicted in FIG. 1A may be associated with temperature sensor 18a. For example, coupling information from coupling sensor 16a may be used to qualify information from temperature sensor 18a. Information from temperature sensor 18a may be deemed useful and/or reliable based on the information from coupling sensor 16a. For example, information from temperature sensor 18a may be discarded based on poor and/or weak coupling between coupling sensor 16a and subject 12, as may be conveyed through coupling information from coupling sensor 16a. The relative position of coupling sensor 16a in relation to temperature sensor 18a as depicted in FIG. 1A (near the lower section on the right-hand side of temperature sensor 18a) is merely exemplary and not intended to be limiting in any way.

The view of body of engagement 14 is partially obscured in FIG. 1A by subject 12. FIG. 1B depicts the same body of engagement 14 (and the same measuring system 10) as depicted in FIG. 1A without subject 12 obscuring the view. Body of engagement 14 may include multiple temperature sensors 18 and multiple coupling sensors 16. The sensors depicted in FIG. 1B may be arranged to form a set, pattern, matrix, grid, and/or other predetermined shape. As depicted in FIG. 1, the sensors of measuring system 10 may be arranged in multiple diagonal lines.

In some embodiments, measuring system 10 includes one or more vital sign state adjustment elements, such as thermal adjust elements 32, configured to adjust one or more vital sign states of subject 12, such as adjusting one or more temperatures of subject 12. Thermal adjustment elements 32 may include one or more heating elements 34 and/or one or more cooling elements 36. In some embodiments, an individual thermal adjustment element 32 may be configured to either heat or cool (at least a region and/or part of) subject 12. In some embodiments, one or more thermal adjustment elements 32 may be associated with one or more coupling sensors 16. For example, as depicted in FIG. 1C, coupling sensor 16b may be associated with cooling element 36a, e.g. based on proximity. In some embodiments, the same individual coupling sensor 16 may be associated with both a temperature sensor 18 and a thermal adjustment element 32. Resulting signals or information from any sensors may be transmitted to processor 110, user interface 120, electronic storage 130, and/or other components of measuring system 10. This transmission may be wired and/or wireless.

By way of illustration, FIG. 1C illustrates another embodiment of the measuring system described in this disclosure, this embodiment depicted as measuring system 10a that includes body of engagement 14a. Measuring system 10a of FIG. 1C may include substantially the same components and functionality as attributed to measuring system 10 of FIG. 1B, except for the number, placement, and type of some of the sensors used. Additionally, as depicted in FIG. 1C, measuring system 10a and body of engagement 14a may include one or more thermal elements 32, for example multiple heating elements 34 and multiple cooling elements 36. As used herein, a generic reference to a heating element or a reference to multiple heating elements may use the term "heating element(s) 34," or variations thereof using the reference numeral "34," whereas a specific individual heating element may be referred to by appending a character to that reference numeral, *e.g.* "heating element 34a", depicted in FIG. 1C. Likewise, FIG. 1C depicts multiple cooling elements 36 as well as a specific cooling element referred to as cooling element 36a.

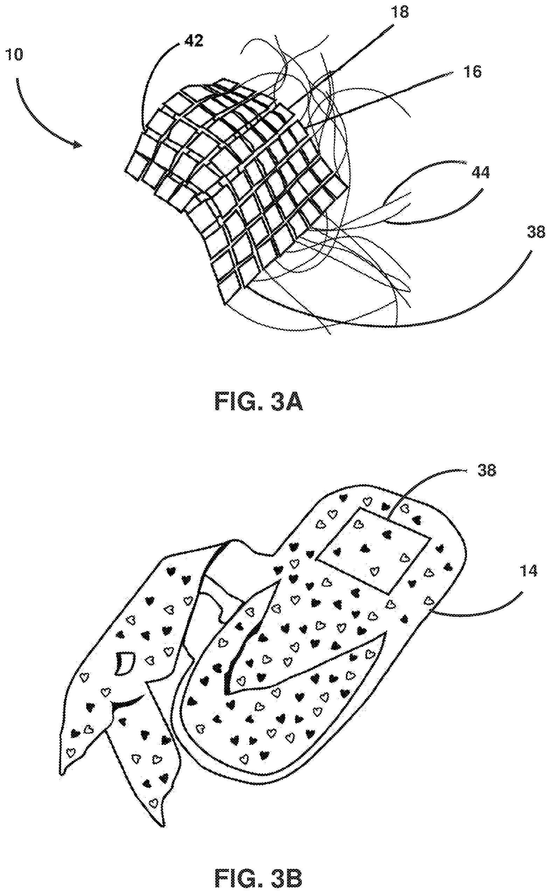

By way of non-limiting example, FIGs. 3A-3B illustrate a measuring system 10 for non-invasive determination of one or more temperatures of a subject 12. FIG. 3A illustrates a temperature matrix 38 comprising of interconnected coupling sensors 16 and temperature sensors 18 configured to determine the temperature of a subject 12. The temperature matrix 38 may comprise individual elements 40. Each element 40 may include one or more temperature sensors 18 and/or one or more coupling sensors 16. The temperature sensors 18 may comprise of one or more zero-heat-flux temperature sensors 20. Each coupling sensor 16 and/or temperature sensor 18 in temperature matrix 38 may be arranged in electrical connection with the other coupling sensors 16 and/or temperature sensors 18 in the temperature matrix 38. Such electrical connection may be provided by wires 42 positioned between the coupling sensors 16 and/or temperature sensors 18. The temperature matrix 38 may be arranged in electrical connection with processor 110 facilitated by wires 44. Such electrical connections may also be effectuated by way of wireless electronic communication between the coupling sensors 16 and/or temperature sensors 18 of the temperature matrix 48 and processor 110. Each coupling sensor 16 and/or temperature sensor 18 may communicate with processor 110 individually or temperature matric 38 may communicate with the processor 110, aggregating data from the sensors. Wireless communication may be achieved using one or more additional components.

FIG. 3B illustrates a body of engagement 14 configured to engage with and/or support a subject 12. FIG. 3B specifically illustrates a carrier, or nest, for a neonate configured to maintain appropriate positioning and flexion of a neonate. The temperature matrix 38 may be integrated into the body of engagement 14, such that the temperature matrix 38 is positioned below the fabric in direct connection with the subject 12 to provide non-invasive temperature measurements.

Referring to measuring system 10 of FIG. 2 (and/or measuring system 10a, as used interchangeably in reference to FIG. 2), measuring system 10 may include electronic storage 130 comprising electronic storage media that electronically stores information. The electronic storage media of electronic storage 130 includes one or both of system storage that is provided integrally (*i.e.,* substantially non-removable) with measuring system 10 and/or removable storage that is connectable to measuring system 10 via, for example, a port (*e.g*., a USB port, a FireWire port, *etc*.) or a drive (*e.g*., a disk drive, *etc*.). Electronic storage 130 may include one or more of optically readable storage media (*e.g*., optical disks, *etc*.), magnetically readable storage media (*e.g*., magnetic tape, magnetic hard drive, floppy drive, *etc*.), electrical charge-based storage media (*e.g*., EEPROM, RAM, *etc*.), solid-state storage media (*e.g*., flash drive, *etc*.), and/or other electronically readable storage media. Electronic storage 130 stores software algorithms, information determined by processor 110, information received via user interface 120, and/or other information that enables measuring system 10 to function properly. For example, electronic storage 130 may record or store (a set of) one or more temperatures and/or parameters derived from output signals measured (*e.g*. over time) by one or more sensors (as discussed elsewhere herein), and/or other information. Electronic storage 130 may be a separate component within measuring system 10, or electronic storage 130 may be provided integrally with one or more other components of system 10 (*e.g*., processor 110).

Referring to FIG. 2, measuring system 10 may include user interface 120 configured to provide an interface between measuring system 10 and a user (*e.g*., user 108, a caregiver, a therapy decision-maker, *etc*.) through which the user can provide information to and receive information from measuring system 10. This enables data, results, and/or instructions and any other communicable items, collectively referred to as "information," to be communicated between the user and measuring system 10. Examples of interface devices suitable for inclusion in user interface 120 include a keypad, buttons, switches, a keyboard, knobs, levers, a display screen, a touch screen, speakers, a microphone, an indicator light, an audible alarm, and a printer. Information may *e.g.* be provided to user 108 by user interface 120 in the form of auditory signals, visual signals, tactile signals, and/or other sensory signals.

By way of non-limiting example, in certain embodiments, user interface 120 includes a radiation source capable of emitting light. The radiation source includes one or more of an LED, a light bulb, a display screen, and/or other sources. User interface 120 may control the radiation source to emit light in a manner that conveys information to, *e.g*., user 108 related to, *e.g*., a breaching of a predetermined temperature threshold by subject 12.

It is to be understood that other communication techniques, either hardwired or wireless, are also contemplated herein as user interface 120. For example, in one embodiment, user interface 120 is integrated with a removable storage interface provided by electronic storage 130. In this example, information is loaded into measuring system 10 from removable storage (*e.g*., a smart card, a flash drive, a removable disk, *etc*.) that enables the user(s) to customize the implementation of measuring system 10. Other exemplary input devices and techniques adapted for use with measuring system 10 as user interface 120 include, but are not limited to, an RS-232 port, RF link, an IR link, modem (telephone, cable, Ethernet, internet or other). In short, any technique for communicating information with measuring system 10 is contemplated as user interface 120.

Referring to FIG. 2, processor 110 is configured to provide information processing capabilities in measuring system 10. As such, processor 110 includes one or more of a digital processor, an analog processor, a digital circuit designed to process information, an analog circuit designed to process information, and/or other mechanisms for electronically processing information. Although processor 110 is shown in FIG. 2 as a single entity, this is for illustrative purposes only. In some embodiments, processor 110 includes a plurality of processing units.

As is shown in FIG. 2, processor 110 is configured to execute one or more computer program modules. The one or more computer program modules include one or more of a coupling module 111, a vital sign state determination module 112, a quality control module 113, a tracking module 114, a map module 115, a target module 116, a control module 117, and/or other modules. Processor 110 may be configured to execute modules 111-117 by software; hardware; firmware; some combination of software, hardware, and/or firmware; and/or other mechanisms for configuring processing capabilities on processor 110.

It should be appreciated that although modules 111-117 are illustrated in FIG. 2 as being co-located within a single processing unit, in implementations in which processor 110 includes multiple processing units, one or more of modules 111-117 may be located remotely from the other modules. The description of the functionality provided by the different modules 111-117 described below is for illustrative purposes, and is not intended to be limiting, as any of modules 111-117 may provide more or less functionality than is described. For example, one or more of modules 111-117 may be eliminated, and some or all of its functionality may be provided by other ones of modules 111-117. Note that processor 110 may be configured to execute one or more additional modules that may perform some or all of the functionality attributed below to one of modules 111-117.

Sensors in this disclosure may be configured to generate output signals in an ongoing manner, *e.g.* throughout the day. This may include generating signals intermittently, periodically (*e.g.* at a sampling rate), continuously, continually, at varying intervals, and/or in other ways that are ongoing during at least a portion of period of a day, week, month, or other duration. The sampling rate may be about 0.001 second, 0.01 second, 0.1 second, 1 second, about 10 seconds, about 1 minute, and/or other sampling rates. It is noted that multiple individual sensors may operate using different sampling rates, as appropriate for the particular output signals and/or (frequencies related to particular) parameters derived therefrom. For example, in some embodiments, the generated output signals may be considered as a vector of output signals, such that a vector includes multiple samples of information conveyed related to one or more temperatures of subject 12. Different temperatures may be related to different vectors. A particular temperature determined in an ongoing manner from a vector of output signals may be considered as a vector of that particular temperature.

Coupling module 111 of measuring system 10 in FIG. 2 is configured to determine coupling levels for one or more sensors of measuring system 10, including but not limited to one or more coupling sensors 16, one or more temperature sensors 18, one or more zero-heat-flux temperature sensors 20, and/or other sensors. As used herein, the term "coupling level" may refer to coupling strength (*e.g.* of electrical signals), and/or signal strength (*e.g.* of electrical signals). In some embodiments, coupling levels may be based on pressure levels, capacitive levels, and/or other types of levels and/or combinations thereof that may indicate whether (and/or to what extent) the output signal from a sensor should be deemed reliable. Alternatively, and/or simultaneously, in some embodiments, a coupling level may indicate whether the output signal from a sensor should be discarded, *e.g.* in favor of stronger and/or more reliable signals from other sensors.

In some embodiments, coupling module 111 may be configured to determine individual coupling levels for individual temperature sensors 18. In some embodiments, determinations by coupling module 111 may be based on one or more coupling signals generated by coupling sensors 16. For example, a coupling level for temperature sensor 18a may be based on coupling information from coupling sensor 16a. In some embodiments, individual temperature sensors 18 may be associated with individual coupling sensors 16, and/or *vice versa.* In some embodiments, information from an individual temperature sensor 18 may be weighted according to the coupling levels of multiple nearby coupling sensors 116. The coupling level for an individual temperature sensor 18 may change over time, for example between measurements taken of individual coupling sensors 116. Changes in coupling levels over time may, for example, be caused by movement of subject 12. Coupling levels from coupling sensors 16 may be ordered, ranked, and/or otherwise compared to coupling levels from one or more other coupling sensors. For example, coupling levels from coupling sensors 116 within a predetermined distance of each other and/or another sensor may be compared with each other and/or with one or more thresholds. Coupling levels from coupling sensors 16 may be compared based on the output signals being generated within the same period, duration, and/or window. By way of non-limiting example, in some embodiments coupling sensors 16 may be configured to generate output signals at a sampling rate of 1 second per measurement. Coupling module 111 may be configured to determine coupling levels for some or all coupling sensors 16 at the same or similar sampling rate such that changing coupling levels may be reevaluated at the same or similar sampling rate to determine whether to use or discard corresponding temperature measurements from associated temperature sensors 18.

Vital sign state determination module 112 of measuring system 10 in FIG. 2 is configured to determine one or more vital sign states of subject 12. As illustrated in the figures, and by way of example only, vital sign state determination module 112 may be configured to determine one or more temperatures of subject 12. The temperatures may include one or more peripheral temperatures at various locations, one or more core temperatures at or near different parts of the body, and/or other temperatures. In some embodiments, vital sign state determination module 112 may be configured to determine multiple temperatures and/or multiple types of temperatures of subject 12, including but not limited to one or more peripheral temperatures and/or one or more core temperatures. Determinations by vital sign state determination module 112 may be based on one or more output signals from one or more temperature sensors 18, one or more coupling signals from one or more coupling sensors 16, and/or one or more coupling levels determined by coupling module 111, one or more determinations by quality control module 113, one or more determinations by map module 115, and/or any combination thereof. For example, output signals from temperature sensors that correspond to a low coupling reliability index (*e.g.* compared to a coupling reliability index threshold and/or to coupling reliability indexes of other sensors) may be discarded, for example in favor of output signals from other temperature sensors that correspond to a high or higher coupling reliability index (*e.g.* compared to the same or a different coupling reliability index threshold and/or to coupling reliability indexes of other sensors). The coupling reliability index determined by quality control module 113 may be further configured to account for the weight of subject 12 without necessitating recalibration of measuring system 10. Coupling levels determined by coupling module 111 may be higher for a subject that is heavier compared to a subject that is lighter.

Movements or displacements of subject 12 away from temperature sensors 18 have been found to cause poor temperature readings. Additionally, the presence of too many layers of material between temperature sensors 18 and subject 12 also cause poor temperature readings. Poor temperature readings may cause temperature monitoring devices to sound false alarms indicating that the core temperature of subject 12 is either too hot or too cold. False alarms cause inefficiencies in medical settings, such as hospital wards and ICU facilities, and also reduce confidence in the temperature measuring devices. Coupling reliability index module 113 of measuring system 10 in FIG. 2 is configured to determine a coupling reliability index based on the coupling levels for the individual ones of the temperature sensors 18 as determined by coupling module 111. The coupling reliability index may indicate the reliability of the determined temperature for individual ones of the multiple temperature sensors 18. The coupling reliability index may indicate the reliability of the determined multiple temperatures of subject 12 as determined by vital sign state determination module 112. The coupling reliability index may indicate that the temperature of subject 12, as detected by one or more temperature sensors 18, is reliable or unreliable based on the coupling level determined by coupling module 111. Coupling reliability index module 113 may be configured to provide an indication of one or more indicia of coupling reliability. By way of non-limiting examples, the one or more indicia of coupling reliability may include a notification that the coupling reliability is reliable or unreliable, include a numerical graded scheme providing a notification of the level of reliability, include a color scheme providing a notification of the level of reliability, and/or other notifications of the level of reliability of the temperature determined by the temperature determination module 113.

In some embodiments, coupling reliability index module 113 may be configured to determine the most likely temperature of subject 12 based upon the determined coupling reliability indexes determined for the multiple temperature sensors 18.

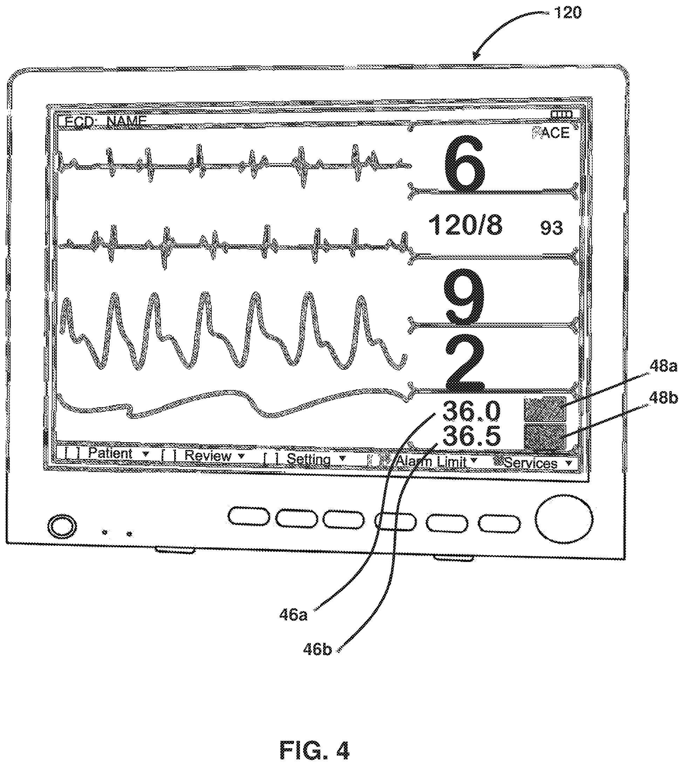

FIG. 4 illustrates an example of user interface 120 for use with measuring system 10 for non-invasive vital sign state measurements of subject 12. For example, the user interface 120 may be configured to facilitate display of one or more temperature measurements 46a, 46b of the subject 12. User interface 120 may also be configured to facilitate display of other vital sign states, such as heart rate, blood pressure and/or other vital sign states of subject 12. User interface 120 may be further configured to display an indication of one or more reliability indexes 48a, 48b as determined by quality control module 113. Such reliability indexes may relate to any and/or all of the vital sign states displayed on user interface 120. For example, temperature measurement 46a may be the temperature of subject 12 measured at a first set of one or more temperature sensors 18 at a first location of the subject 12. Temperature measurement 46b may be the temperature of a subject 12 measured at a second set of one or more temperature sensors 18 at a second location of the subject 12. Indication of reliability index 48a provides an indication of the reliability of temperature measurement 46a based upon coupling level(s) for the first set of temperature sensors 18 at a first location of the subject 12. Indication of reliability index 48b provides an indication of the reliability of temperature measurement 46b based upon coupling level(s) for the second set of temperature sensors 18 at a second location of the subject 12. By way of non-limiting example, the indicia of reliability index 48a may be a color indicating that the temperature measurement 46a is reliable, due to the relatively high coupling levels at the first location compared to the coupling levels at the second location which causes the indicia of reliability index 48b to be a color indicating that the temperature measurement 46b is less reliable.

In some embodiments, measuring system 10 in FIG. 2 may comprise a tracking module 114 configured to track changed in the coupling reliability index of individual vital state sensors over time. Tracking module 114 may be configured to track changes in the span of about 10 minutes, about an hour, about 2 hours, about 4 hours, about 8 hours, about 12 hours, about 24 hours, about 48 hours, about 72 hours, about a week, about a month, about 2 months, and/or other amounts of time. Relatively slow changes in coupling reliability index (compared to the sampling rate) may indicate a change in position of the subject 12, or a change in the functionality of one or more of the coupling sensors 16 and/or temperature sensors 18 indicating that the coupling sensors 16 and/or temperature sensors 18 have a fault. Tacking module 114 may be configured to track the coupling reliability index and/or coupling levels over time for sets of one or more temperature sensors 18 and determine whether any detected change is a result of movement of the subject across the temperature sensors 18. For example, a gradual reduction in the coupling levels and/or coupling reliability index for a first set of one or more temperature sensors 18 accompanied by a gradual increase in the coupling levels and/or coupling reliability index for an adjacent second set of one or more temperature sensors may indicate the gradual movement of the subject 12 across the temperature sensors.

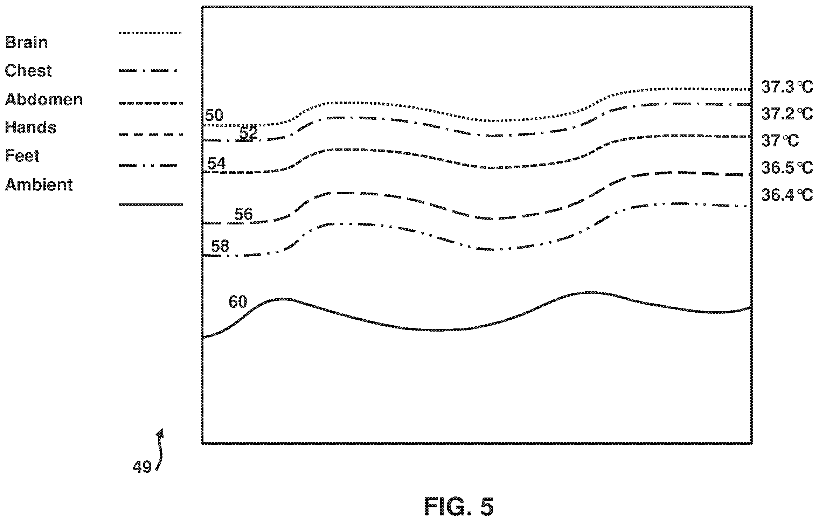

In some embodiments, temperature sensors 16 may include one or more zero-heat-flux temperature sensors 20. Vital sign state determination module 112 may be configured to determine one or more core temperatures of subject 12 based on output signals generated by zero-heat-flux temperature sensors 20. Alternatively, and/or simultaneously, one or more determined core temperatures of subject 12 may further be based on one or more coupling levels determined by coupling module 111. For example, a particular core temperature may be based on a coupling level for zero-heat-flux temperature sensor 20a, which may be based on coupling information from coupling sensor 116b. Vital sign state determination module 112 may be configured to determine multiple temperatures of subject 12 over time. By way of non-limiting example, FIG. 4 illustrates a graph 49 including multiple temperatures (in °C) measured over time (along the X-axis), including temperatures for the brain (50), chest (52), abdomen (54), hands (56), feet (58), and ambient temperature (60). By way of non-limiting example, brain temperature 50 may be a core temperature and feet temperature 58 may be a peripheral temperature. In some embodiments, user interface 120, of FIG. 4, may be configured to facilitate display of the graph 49 depicted in FIG. 5. User interface 120 may be configured to display multiple temperatures of the subject 12. For example, temperature measurement 46a may be the measured core temperature, such as brain temperature 50 or abdomen temperature 54, and temperature measurement 46b may be the measured peripheral temperature, such as feet temperature 58 or hands temperature 56.

In some embodiments, vital sign state determination module 112 may be configured to determine one or more temperatures of subject 12 without using or needing coupling information. For example, determinations by vital sign state determination module 112 may be based on one or more of positional information (described elsewhere herein), and/or a temperature map of subject 12 (*e.g.* determined by map module 115).

In some embodiments, measuring system 10 may include one or more sensors configured to generate output signals conveying positional information of subject 12. Positional information of subject 12 may include information about the relative position of subject 12 (and/or one or more body parts of subject 12) as compared to one or more of measuring system 10, body of engagement 14, a support structure in which subject 12 has been placed, an incubator, a crib, all or part of a NICU, cradle, nest, and/or another object. In some embodiments, positional information may be derived from and/or based on coupling information. In some embodiments, positional information may be derived from (*e.g.* deduced from) one or more temporal variations of one or more temperatures and/or variations of the temperature map of subject 106 over time, for example in conjunction with a (parameterized) model that does not use coupling information. Alternatively, and/or simultaneously, in some embodiments, positional information may be derived from and/or based on information conveyed by one or more image sensors. For example, positional information may be based on information from a (video and/or photography) camera. In some embodiments, positional information may be determined by coupling module 111. Alternatively, and/or simultaneously, in some embodiments, positional information may be derived from and/or based on information conveyed by one or more temperature sensors, *e.g.* in combination with coupling information. For example, positional information may be based on (*e.g.* derived, deduced, and/or inferred from) a temperature map of a subject, *e.g.* as determined by map module 115.

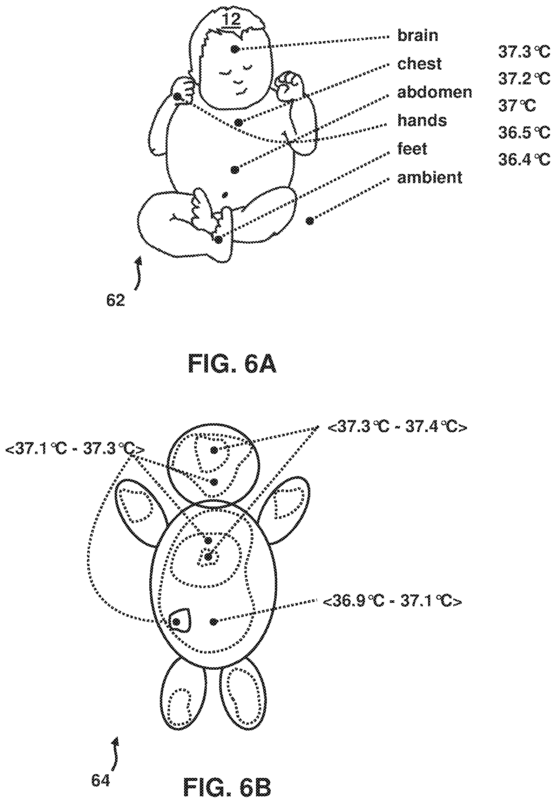

Map module 115 of measuring system 10 in FIG. 2 is configured to determine and/or construct a vital sign state profile of subject 12 based on vital sign states determined by vital sign state determination module 112 of subject 12. As an example, a vital sign state profile of subject 12 may include a temperature map of subject 12 based on temperatures determined by vital sign state determination module 112 and/or positional information of subject 12. As used herein, the term "temperature map" may be used interchangeably with the terms "temperature profile" and "graphical temperature representation". For example, a temperature map may depict an image subject 12 combined with information about different relevant temperatures. By way of non-limiting illustration, FIG. 6A illustrates a temperature map 62 of subject 12. In some embodiments, the image used in temperature map 62 may be an actual representation (*e.g.* a photograph) of subject 12. In some embodiments, the image used in temperature map 62 may be a real-time representation (*e.g.* a video image) of subject 12. Temperature map 62 may, by way of non-limiting example, include the same or similar temperatures as depicted in FIG. 5, including temperatures for the brain, chest, abdomen, hands, feet, and ambient temperature. By way of example, the end temperatures (*i.e.* the right-most temperatures depicted) from graph 49 (FIG. 5) are depicted as the current temperatures in temperature map 62 in FIG. 6A. Temperature map 62 may be 2-dimensional or more-then-2-dimensional, for example 3-dimensional.

In some embodiments, a temperature map of subject 12 may be based on a (parameterized) model using multiple determined temperatures of subject 12. Optionally, the model may use coupling information. For example, the model may include coupling reliability index information tracked over time by tracking module 114 to determine the position of subject 12. The model may facilitate display of a temperature map of subject 12 on user interface 120 of FIG. 4. Optionally, the model may use positional information of subject 12, *e.g.* for embodiments in which positional information is determined independently of a temperature map. In some embodiments, a temperature map may be inferred from multiple determined temperatures of subject 12 and positional information of subject 12.

In some embodiments, a temperature map may depict regions of subject 12 having the same or similar temperature, such as a heat map. Such regions may for example be indicated using different colors. In some embodiments, the image used in temperature map 64 may be an actual representation (*e.g.* a photograph) of subject 12, or a schematic representation (including head, torso, arms, and legs) as depicted in FIG. 6B. This list of body parts is exemplary and not intended to be limiting in any way. By way of non-limiting example, FIG. 6B illustrates a temperature map 64 that depicts regions of subject 12 having similar temperatures. For example, two regions are indicated as having a temperature between 37.3 °C and 37.4 °C, three regions are indicated as having a temperature between 37.1 °C and 37.3 °C, one region is indicated as having a temperature between 36.9 °C and 37.1 °C. The different temperatures (or temperature ranges) may be indicated in a temperature map using different colors. In some embodiments, the image used in temperature map 64 to represent subject 12 may be a real-time 3-dimensional representation of subject 12. By way of non-limiting example, one or both of the two described regions may be core temperatures and one or more of the temperatures and/or regions associated with the extremities of subject 12 may be peripheral temperatures.

As stated previously, the measurement of multiple vital sign states may produce a vital sign state profile of subject 12. The description herein with reference to the measurement of temperatures of subject 12 can be applied to the measurement of any vital sign of subject 12 and also to multiple vital sign states of subject 12 at the same time to provide a holistic view of the vital signs of subject 12.

Tracking module 114 of system 10 in FIG. 2 may be further configured to track changes in one or more temperatures over time. Tracking module 114 may be configured to track changes in the span of about 10 minutes, about an hour, about 2 hours, about 4 hours, about 8 hours, about 12 hours, about 24 hours, about 48 hours, about 72 hours, about a week, about a month, about 2 months, and/or other amounts of time. Relatively slow changes in temperature (compared to the sampling rate) may indicate a change in a medical condition that might be noteworthy. For example, a particular temperature (*e.g.* determined by vital sign state determination module 112) may rise or fall outside an acceptable and/or preferred range for such a temperature. In some embodiments, tracking module 114 may be configured to determine whether a difference between two temperatures increases or decreases over time, and/or whether such a change falls outside an acceptable and/or preferred range for such a difference. For example, tracking module 114 may be configured to determine whether the peripheral temperature of one or both hands differs more than a predetermined maximum difference threshold from the temperature of the brain. For example, tracking module 114 may be configured to determine whether the peripheral temperatures of the extremities are more than a predetermined maximum difference threshold apart from each other.

In some embodiments, tracking module 114 may be configured to determine whether one or more temperatures and/or changes in temperatures indicate significant information pertinent to diagnostic purposes, as described elsewhere herein. System 10 may be configured to measure other patient-specific parameters as needed to support the process of such determinations, including but not limited to physiological parameters, respiratory parameters, and/or any other medically relevant parameters and/or combinations thereof. For example, a particular predetermined combination of a change in heart rate, a change in respiratory rate, and a change in one or more temperatures may indicate a particular medical condition or emergency that may be noteworthy to a user and/or caregiver. As used herein, the term "predetermined" may refer to a determination that has been made prior to usage of system 10 on a particular subject. For example, a programmed relation, value, or threshold may be referred to as predetermined. In some embodiments, tracking module 114 may be configured to notify and/or alert a user or caregiver responsive to one or more determinations (described in this disclosure) having been made.

Target module 116 is configured to obtain and/or determine one or more target temperatures and/or target temperature ranges for subject 12. For example, the one or more target temperatures may be specific to the type *(e.g.* core, peripheral, or other) and/or location of the measurements (*e.g.* which body part, organ, area, and/or region of subject 12). One or more target temperatures and/or target temperature ranges may be recommended by one or more medical professionals as being desirable for subject 12. Determined temperatures (*e.g.* by vital sign state determination module 112) may be compared to one or more target temperatures and/or target temperature ranges. For example, a target temperature range for the brain temperature may be between 37.2 °C and 37.5 °C. Responsive to a determination that a brain temperature falls outside of the corresponding target temperature range, system 10 may be configured to (attempt to) adjust the relevant temperature of subject 12, as described elsewhere herein.

Control module 117 of measuring system 10 in FIG. 2 is configured to control one or more thermal adjustment elements 32. In some embodiments, control module 117 may be configured to control one or more thermal adjustment elements 32 in accordance with a therapy regimen. In some embodiments, control module 116 may be configured to control one or more thermal adjustment elements 32 to adjust one or more of the determined temperatures (*e.g.* as determined by vital sign state determination module 112). In some embodiments, control module 117 may be configured to control one or more thermal adjustment elements 32 based on one or more comparisons between a determined temperature and a target temperature (and/or target temperature range). In some embodiments, control module 117 may be configured to control one or more thermal adjustment elements 32 in accordance with one or more determined target temperatures and/or target temperature ranges (*e.g.* as determined by target module 116). For example, responsive to a comparison between a target temperature and a corresponding determined temperature, control module 117 may be configured to increase or decrease a particular body part, organ, area, and/or region of subject 12. This may be referred to as heating or cooling, respectively. For example, heating may be accomplished using one or more heating elements 34; cooling may be accomplished using one or more cooling elements 36. Selection of one or more particular thermal adjustment elements 32 may depend on locality and/or proximity of the corresponding temperature sensor(s) 18. Alternatively, and/or simultaneously, in some embodiments, selection of one or more particular thermal adjustment elements 32 may depend on coupling levels as determined for nearby coupling sensors 11641, by virtue of the notion that weak electrical and/or thermal coupling may affect the efficacy of a thermal adjustment element at the same or similar location (*e.g.* for a thermal adjustment element 32 located close to a coupling sensor 16).

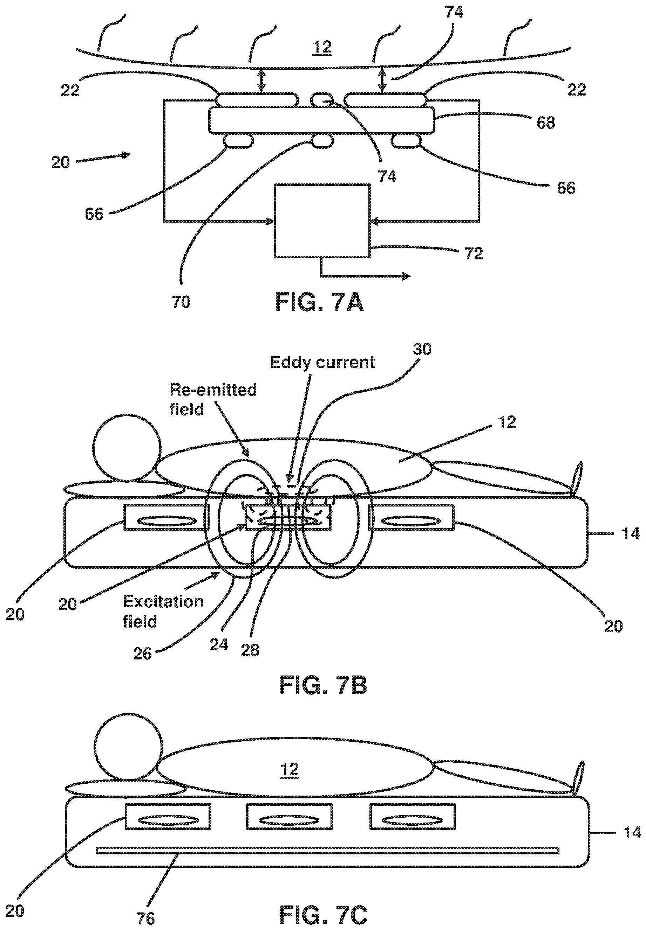

FIGs. 7A-7B-7C illustrate measuring systems 10 for non-invasive determination of one or more temperatures of a subject 12, in accordance with one or more embodiments. Referring now to FIG. 7A, shown is a zero-heat-flux sensor 20 positioned within a body of engagement 14. Subject 12 is placed over the zero-heat-flux sensor 20. The zero-heat-flux sensor 20, may comprise thermo-isolator 68. Thermo-isolator 68 may be made from a thermo-isolating material and may also be the support structure, or carrier, for some or all of the components of zero-heat-flux sensor 20. The zero-heat-flux sensor 20 may comprise temperature sensors 70. Temperature sensor 70 may be a thermistor and/or other temperature sensor. In some embodiments, a first temperature sensor 70 may be positioned on a first side of thermo-isolator 68, and a second temperature sensor 70 may be positioned on a second side of thermo-isolator 68. Heating elements 66 may be positioned on the first side and/or second side of thermo-isolator 68. Heating element 66 may be a resistor and/or other heating element.

In some embodiments, as shown in FIG. 7A, coupling sensors 16 of FIGs. 1A-1B-1C may be electrodes 22 adapted to form the capacitive coupling with subject 12. The thermo-isolator 68 may have a uniform temperature grade across it, and may be configured to be a thermal equalizer for the zero-heat-flux temperature sensor 20. In such systems, heating elements 66 may also be electrodes 22 to form the capacitive coupling with subject 12, obviating the need for additional separate electrodes 22. In cases where coupling sensors 16 being electrodes 22 are configured to generate analog coupling signals conveying coupling information with subject 12, the zero-heat-flux sensor 20 may also comprise a capacitance-to-digital convertor 72 adapted to convert the analog signal generated by electrodes 22 into digital format information to be processed by processor(s) 110. The signal generated by electrodes 22 may convey electrical coupling information with the subject.

Capacitance formed by electrodes 22 via the body 12 is inversely proportional to the distance 74, or average density of the fabric, between the body and the electrodes. When distance 74 increases, electrodes 22 generate coupling signals conveying electrical coupling information with subject 12 indicating a reducing level of coupling for the zero-heat-flux temperature sensor 20.

In some embodiments, zero-heat-flux temperature sensor 20 may be combined with other zero-heat-flux temperature sensor to form a matrix, such as thermal matrix 40 illustrated in FIG. 3A. In such embodiments, electrodes 22, also being heating elements 66, may be floating electrodes in order to homogenously distribute the heat across the thermal matrix 40.

Electrodes 22, being capacitive sensors 16 of FIG. 2, may also be adapted to detect biological electric fields such as those produced by heart activity, lung activity, or other organ activity. In this manner, electrodes 22 may also be used to measure ECG, heart rate, respiration, and/or other biological processes in subject 12. Electrodes 22, also functioning as capacitive sensors 16 will generate signals conveying coupling information for those biological electric fields.

Referring now to FIG. 7B, shown is one or more zero-heat-flux sensors 20 using magnetic induction to determine the coupling reliability index between body 12 and temperature sensors 18. The coupling sensors 16, as shown in FIGs. 1A-1B-1C, comprise of a first set of one or more excitation coils 24 configured to generate a first set of one or more magnetic fields 26 and to detect a second set of one or more magnetic fields 30 generated by subject 12, from eddy currents generated by subject 12, in response to the generation of first set of one or more magnetic fields 26. In other embodiments a second set of one or more excitation coils 28 configured to detect second set of one or more magnetic fields 30, instead of first set of one or more excitation coils 26 both generating and detecting magnetic fields. Heater element 66, as shown in FIG. 7A, may be an excitation coil. In this manner heater element 66 may also be first set of one or more excitation coils 26. In such embodiments, quality control module 113 is configured to determine the coupling reliability index based on the capacitance formed between first set of one or more excitation coils 24 and subject 12.

Referring now to FIG. 7C, shown is one or more zero-heat-flux sensors 20 using pressure sensors to determine the coupling reliability index between body 12 and temperature sensors 18. Pressure foil 76 may be positioned within body of engagement 14 on the opposing side of the one or more zero-heat-flux temperature sensors 20 to subject 12. In other embodiments, pressure foil 76 may be a set of one or more pressure foils integrated with each of the one or more zero-heat-flux-sensors 20. In such embodiments, pressure foil 76 is configured to generate signals conveying pressure information, coupling module 111 is configured to determine coupling levels for individual ones of the temperature sensors based on the pressure information conveyed in the signals generated by pressure foil 76. Quality control module 113 is configured to determine a relatively high coupling reliability index for the temperature sensor 18 located at a high-pressure region on pressure foil 76, compared to other temperature sensors at low-pressure regions on pressure foil 76.

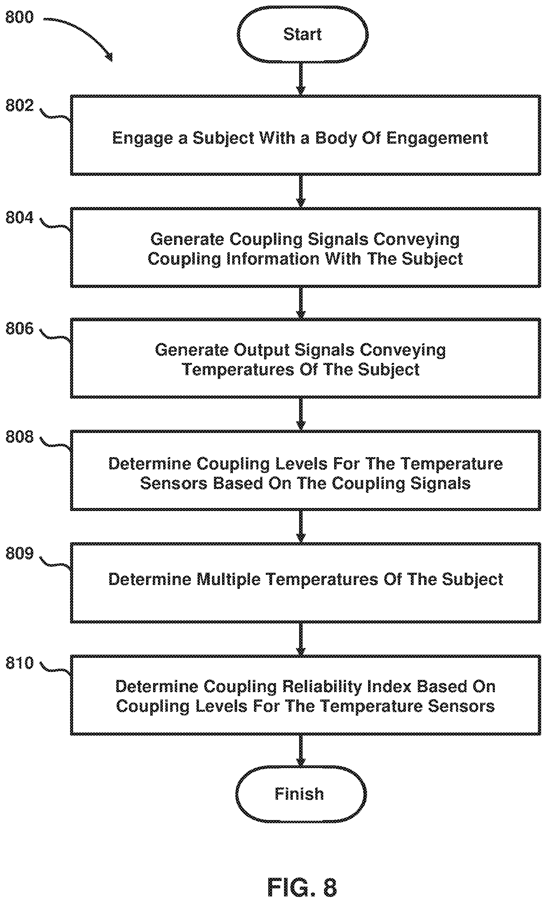

FIG. 8 illustrates a specific method 800 to determine one or more temperatures of a subject. One of ordinary skill in the art would realize and understand that method 800 is an exemplary embodiment of the disclosed non-invasive method for determining one or more vital sign states of a subject and is not intended to be limiting. The operations of method 800 presented below are intended to be illustrative. In certain embodiments, method 800 may be accomplished with one or more additional operations not described, and/or without one or more of the operations discussed. Additionally, the order in which the operations of method 800 are illustrated in FIG. 8 and described below is not intended to be limiting.

In certain embodiments, method 800 may be implemented in one or more processing devices (*e.g*., a digital processor, an analog processor, a digital circuit designed to process information, an analog circuit designed to process information, and/or other mechanisms for electronically processing information). The one or more processing devices may include one or more devices executing some or all of the operations of method 800 in response to instructions stored electronically on an electronic storage medium. The one or more processing devices may include one or more devices configured through hardware, firmware, and/or software to be specifically designed for execution of one or more of the operations of method 800.

At an operation 802, a subject engages with a body of engagement. In some embodiments, operation 802 is performed by a body of engagement the same as or similar to body of engagement 14 (shown in FIG. 1A and described herein).

At an operation 804, coupling signals are generated conveying coupling information with the subject at or near a point of engagement between the subject and the body of engagement. In some embodiments, operation 804 is performed by coupling sensors the same as or similar to coupling sensors 16 (shown in FIG. 1A and described herein).

At an operation 806, output signals are generated conveying temperatures of the subject at or near a point of engagement between the subject and the body of engagement. In some embodiments, operation 806 is performed by temperature sensors the same as or similar to temperature sensors 18 (shown in FIG. 1A and described herein).

At an operation 808, coupling levels are determined for individual ones of the temperature sensors based on the coupling signals. In some embodiments, operation 808 is performed by a coupling module the same as or similar to coupling module 111 (shown in FIG. 2 and described herein).

At an operation 810, multiple temperatures of the subject are determined based on the output signals and the determined coupling levels. In some embodiments, operation 810 is performed by a vital sign state determination module the same as or similar to vital sign state determination module 112 (shown in FIG. 2 and described herein).

At an operation 812, a coupling reliability index is determined, based on the coupling levels for the individual ones of the temperature sensors determined at operation 808. In some embodiments, operation 812 is performed by a temperature determination module the same as or similar to quality control module 113 (shown in FIG. 2 and described herein).

In the claims, any reference signs placed between parentheses shall not be construed as limiting the claim. The word "comprising" or "including" does not exclude the presence of elements or steps other than those listed in a claim. In a device claim enumerating several means, several of these means may be embodied by one and the same item of hardware. The word "a" or "an" preceding an element does not exclude the presence of a plurality of such elements. In any device claim enumerating several means, several of these means may be embodied by one and the same item of hardware. The mere fact that certain elements are recited in mutually different dependent claims does not indicate that these elements cannot be used in combination.

Although this description includes details for the purpose of illustration based on what is currently considered to be the most practical and preferred embodiments, it is to be understood that such detail is solely for that purpose and that the disclosure is not limited to the disclosed embodiments, but, on the contrary, is intended to cover modifications and equivalent arrangements that are within the scope of the appended claims. For example, it is to be understood that, to the extent possible, one or more features of any embodiment are contemplated to be combined with one or more features of any other embodiment.

## Claims

1. A method of non-invasive determination and control of one or more vital sign states of a subject, the method comprising:
engaging a subject with a body of engagement;
generating, by coupling sensors, coupling signals conveying coupling information with the subject, wherein the coupling sensors are carried by the body of engagement;
generating, by vital sign sensors, output signals conveying vital sign information of the subject, wherein the vital sign sensors are carried by the body of engagement; and
determining coupling levels for individual ones of the vital sign sensors based on the coupling signals;
determining multiple vital sign states of the subject based on the output signals and the determined coupling levels; and,
determining a coupling reliability index based on the coupling levels for the individual ones of the vital sign sensors.

2. The method of claim 1, wherein the coupling reliability index indicates the reliability of the determined multiple vital sign states of the subject.

3. The method of claim 1, wherein the vital sign sensors include a zero-heat-flux temperature sensor wherein generating output signals conveying vital sign information of the subject includes:
creating, by a zero-heat-flux temperature sensor, thermal insulation between the body of engagement and the subject; and,
generating, by the zero-heat-flux temperature sensor, output signals conveying a second temperature of the subject,
wherein determining multiple vital sign states of the subject includes determining a core temperature based on the output signals generated by the zero-heat-flux temperature sensor.

4. The method of claim 1, wherein the coupling sensors include a set of one or more electrodes wherein determining a coupling reliability index comprises:
positioning the set of one or more electrodes adjacent the subject;
forming a capacitance, by the set of one or more electrodes, between the electrodes and the subject; and,
determining the coupling reliability index based on the capacitance formed between the electrodes and the subject.

5. The method of claim 1, wherein the coupling sensors include a first set of one or more excitation coils and a second set of one or more excitation coils, wherein determining a coupling reliability index comprises:
generating, by the first set of one or more excitation coils, a first set of one or more magnetic fields;
causing the subject to generate a second set of one or more magnetic fields in response to an interaction with the first set of one or more magnetic fields;
detecting, by a second set of one or more excitation coils, the second set of one or more magnetic fields generated by the subject;
determining the coupling reliability index based on a difference between the first set of one or more magnetic fields and the second set of one or more magnetic fields.

6. The method of claim 1, further comprising the step of:
tracking changes in the coupling reliability index of individual vital sign sensors over time.

7. A system (10) for non-invasive determination and control of one or more vital sign states of a subject, the system comprising:
means (14) for engaging a subject with a body;
means (16) for generating coupling signals conveying coupling information with the subject, wherein the means for generating coupling signals are carried by the means for engaging the subject with the body;
means (18) for generating output signals conveying vital sign information of the subject, wherein the means for generating output signals are carried by the means for engaging the subject with the body;
means (111) for determining coupling levels for individual ones of the means for generating output signals conveying vital sign information of the subject, based on the coupling signals generated by the means for generating coupling signals;
means (112) for determining multiple vital sign states (46) of the subject based on the output signals from the means for generating output signals conveying vital sign information of the subject and the determined coupling levels; and,
means (113) for determining a coupling reliability index based on the coupling levels for the means for generating output signals conveying vital sign information of the subject.

8. The system of claim 7, wherein the coupling reliability index (48) indicates the reliability of the determined multiple vital sign states of the subject as determined by the means (112) for determining multiple vital sign states (46) of the subject.

9. The system of claim 7, wherein the means for determining multiple vital sign states of the subject includes:
means (68) for creating thermal insulation between the body of engagement and the subject, and
means (20) for generating output signals conveying a first temperature of the subject,
wherein the means for determining multiple vital sign states of the subject is configured to determine a core temperature based on the second temperature.

10. The system of claim 7, wherein the means for generating coupling signals comprises a means (22) for forming a capacitance between the means for forming a capacitance and the subject, and wherein the means for determining the coupling reliability index is based on the capacitance formed between the means for forming a capacitance and the subject.

11. The system of claim 7, wherein the means for generating coupling signals conveying coupling information comprises:
a means (24) for generating a first set of one or more magnetic fields, wherein the first set of one or more magnetic fields cause the subject to generate a second set of one or more magnetic fields in response to an interaction by the first set of one or more magnetic fields with the subject; and,
a means (28) for detecting the second set of one or more magnetic fields generated by the subject;
wherein the means for determining the coupling reliability index based on the capacitance formed between a difference between the first set of one or more magnetic fields and the second set of one or more magnetic fields.

12. The system of claim 11, wherein the means (24) for generating a first set of one or more magnetic fields comprises a first set of one or more excitation coils, and wherein the means (28) for detecting the second set of one or more magnetic fields generated by the subject comprises a second set of one or more excitation coils.

13. The system of claim 7, further comprising:
a means (114) for tracking changes in the coupling reliability index of the means for generating output signals conveying the vital sign information of a subject over time.

14. The system of claim 7, wherein the means for generating coupling signals is carried by the means (14) for engaging the subject with the body, and comprises means for conveying information relating to electrical, thermal and/or magnetic coupling with the subject.

## Patentansprüche

1. Verfahren zum nicht-invasiven Bestimmen und Steuern von einem oder mehreren Vitalparameterzuständen eines Probanden, wobei das Verfahren umfasst:
Inkontaktbringen eines Probanden mit einem Kontaktkörper;
Erzeugen, durch Kopplungssensoren, von Kopplungssignalen, die Kopplungsinformationen mit dem Probanden übermitteln, wobei die Kopplungssensoren von dem Kontaktkörper getragen werden;
Erzeugen, durch Vitalparametersensoren, von Ausgangssignalen, die Vitalparameterinformationen des Probanden übermitteln, wobei die Vitalparametersensoren vom Kontaktkörper getragen werden; und
Bestimmen von Kopplungsniveaus für einzelne der Vitalparametersensoren basierend auf den Kopplungssignalen;
Bestimmen mehrerer Vitalparameterzustände des Probanden basierend auf den Ausgangssignalen und den bestimmten Kopplungsniveaus; und
Bestimmen eines Kopplungszuverlässigkeitsindex basierend auf den Kopplungsniveaus für die einzelnen der Vitalparametersensoren.

2. Verfahren nach Anspruch 1, wobei der Kopplungszuverlässigkeitsindex die Zuverlässigkeit der bestimmten mehreren Vitalparameterzustände des Probanden anzeigt.

3. Verfahren nach Anspruch 1, wobei die Vitalparametersensoren einen Null-Wärmefluss-Temperatursensor beinhalten, wobei das Erzeugen von Ausgangssignalen, die Vitalparameterinformationen des Probanden übermitteln, beinhaltet:
Erschaffen, durch einen Null-Wärmestrom-Temperatursensor, einer Wärmedämmung zwischen dem Kontaktkörper und dem Probanden; und
Erzeugen, durch den Null-Wärmefluss-Temperatursensor, von Ausgangssignalen, die eine zweite Temperatur des Probanden übermitteln,
wobei das Bestimmen mehrerer Vitalparameterzustände des Probanden das Bestimmen einer Kerntemperatur basierend auf den Ausgangssignalen, die durch den Null-Wärmefluss-Temperatursensor erzeugt werden, beinhaltet.

4. Verfahren nach Anspruch 1, wobei die Kopplungssensoren einen Satz von einer oder mehreren Elektroden beinhalten, wobei das Bestimmen eines Kopplungszuverlässigkeitsindex umfasst:
Positionieren des Satzes von einer oder mehreren Elektroden neben dem Probanden;
Bilden einer Kapazität durch den Satz von einer oder mehrerer Elektroden zwischen den Elektroden und dem Probanden; und
Bestimmen des Kopplungszuverlässigkeitsindex basierend auf der zwischen den Elektroden und dem Probanden gebildeten Kapazität.

5. Verfahren nach Anspruch 1, wobei die Kopplungssensoren einen ersten Satz von einer oder mehrerer Erregerspulen und einen zweiten Satz von einer oder mehrerer Erregerspulen beinhalten, wobei das Bestimmen eines Kopplungszuverlässigkeitsindex umfasst:
Erzeugen, durch den ersten Satz von einer oder mehrerer Erregerspulen, eines ersten Satzes von einem oder mehreren Magnetfeldern;
Veranlassen des Probanden, einen zweiten Satz von einem oder mehreren Magnetfelder als Reaktion auf eine Wechselwirkung mit dem ersten Satz von einem oder mehreren Magnetfeldern zu erzeugen;
Erfassen, durch einen zweiten Satz von einem oder mehreren Erregerspulen, des zweiten Satzes von einem oder mehreren Magnetfeldern, die von dem Probanden erzeugt werden;
Bestimmen des Kopplungszuverlässigkeitsindex basierend auf einer Differenz zwischen dem ersten Satz von einem oder mehreren Magnetfeldern und dem zweiten Satz von einem oder mehreren Magnetfeldern.

6. Verfahren nach Anspruch 1, weiter umfassend den Schritt von:
Nachverfolgen von Veränderungen im Kopplungszuverlässigkeitsindex einzelner Vitalparametersensoren im Zeitablauf.

7. System (10) zum nicht-invasiven Bestimmen und Steuern von einem oder mehreren Vitalparameterzuständen eines Probanden, wobei das System umfasst:
Mittel (14) zum Inkontaktbringen eines Probanden mit einem Körper;
Mittel (16) zum Erzeugen von Kopplungssignalen, die Kopplungsinformationen mit dem Probanden übermitteln, wobei die Mittel zum Erzeugen von Kopplungssignalen von den Mitteln zum Inkontaktbringen des Probanden mit dem Körper getragen werden;
Mittel (18) zum Erzeugen von Ausgangssignalen, die Vitalparameterinformationen des Probanden übermitteln, wobei die Mittel zum Erzeugen von Ausgangssignalen durch die Mittel zum Inkontaktbringen des Probanden mit dem Körper getragen werden;
Mittel (111) zum Bestimmen von Kopplungsniveaus für einzelne der Mittel zum Erzeugen von Ausgangssignalen, die Vitalparameterinformationen des Probanden übermitteln, basierend auf den Koppelsignalen, die von den Mitteln zum Erzeugen von Kopplungssignalen erzeugt werden;
Mittel (112) zum Bestimmen mehrerer Vitalparameterzustände (46) des Probanden basierend auf den Ausgangssignalen von den Mitteln zum Erzeugen von Ausgangssignalen, die Vitalparameterinformationen des Probanden und die bestimmten Kopplungsniveaus übermitteln; und
Mittel (113) zum Bestimmen eines Kopplungszuverlässigkeitsindex basierend auf den Kopplungsniveaus für die Mittel zum Erzeugen von Ausgangssignalen, die Vitalparameterinformationen des Probanden übermitteln.

8. System nach Anspruch 7, wobei der Kopplungszuverlässigkeitsindex (48) die Zuverlässigkeit der bestimmten mehreren Vitalparameterzustände des Probanden angibt, wie sie durch die Mittel (112) zum Bestimmen mehrerer Vitalparameter (46) des Probanden bestimmt wird.

9. System nach Anspruch 7, wobei das Mittel zum Bestimmen mehrerer Vitalparameterzustände des Probanden Folgendes beinhaltet:
Mittel (68) zum Erzeugen einer Wärmedämmung zwischen dem Kontaktkörper und dem Probanden, und
Mittel (20) zum Erzeugen von Ausgangssignalen, die eine erste Temperatur des Probanden übermitteln,
wobei das Mittel zum Bestimmen mehrerer Vitalparameterzustände des Probanden konfiguriert ist, um eine Kerntemperatur basierend auf der zweiten Temperatur zu bestimmen.

10. System nach Anspruch 7, wobei das Mittel zum Erzeugen von Kopplungssignalen ein Mittel (22) zum Bilden einer Kapazität zwischen dem Mittel zum Bilden einer Kapazität und dem Probanden umfasst, und wobei das Mittel zum Bestimmen des Kopplungszuverlässigkeitsindex auf der Kapazität basiert, die zwischen dem Mittel zum Bilden einer Kapazität und dem Probanden gebildet wird.

11. System nach Anspruch 7, wobei das Mittel zum Erzeugen von Kopplungssignalen, die Kopplungsinformationen übertragen, umfasst:
ein Mittel (24) zum Erzeugen eines ersten Satzes von einem oder mehreren Magnetfeldern, wobei der erste Satz von einem oder mehreren Magnetfeldern bewirkt, dass der Proband einen zweiten Satz von einem oder mehreren Magnetfeldern als Reaktion auf eine Wechselwirkung des ersten Satzes von einem oder mehreren Magnetfeldern mit dem Probanden erzeugt; und
ein Mittel (28) zum Erfassen des zweiten Satzes von einem oder mehreren Magnetfeldern, die von dem Probanden erzeugt werden;
wobei die Mittel zum Bestimmen des Kopplungszuverlässigkeitsindex auf der Kapazität basiert, die zwischen einer Differenz zwischen dem ersten Satz von einem oder mehreren Magnetfeldern und dem zweiten Satz von einem oder mehreren Magnetfeldern gebildet wird.

12. System nach Anspruch 11, wobei die Mittel (24) zum Erzeugen eines ersten Satzes von einem oder mehreren Magnetfeldern einen ersten Satz von einer oder mehreren Erregerspulen umfassen, und wobei die Mittel (28) zum Erfassen des zweiten Satzes von einem oder mehreren Magnetfeldern, die von dem Probanden erzeugt werden, einen zweiten Satz von einer oder mehreren Erregerspulen umfassen.

13. System nach Anspruch 7, weiter umfassend:
ein Mittel (114) zum Nachverfolgen von Veränderungen im Kopplungszuverlässigkeitsindex des Mittels zum Erzeugen von Ausgangssignalen, die die Vitalparameterinformationen eines Probanden im Zeitablauf übertragen.

14. System nach Anspruch 7, wobei das Mittel zum Erzeugen von Kopplungssignalen von dem Mittel (14) zum Inkontaktbringen des Probanden mit dem Körper getragen wird, und Mittel zum Übermitteln von Informationen im Zusammenhang mit der elektrischen, thermischen und/oder magnetischen Kopplung mit dem Probanden umfasst.

## Revendications

1. Procédé de détermination et de régulation non invasives d'un ou plusieurs états de signes vitaux d'un sujet, le procédé comprenant :
la mise en prise d'un sujet avec un corps de mise en prise ;
la production, par des capteurs de couplage, de signaux de couplage transportant des informations de couplage avec le sujet, dans lequel les capteurs de couplage sont portés par le corps de mise en prise ;
la production, par des capteurs de signes vitaux, de signaux de sortie transportant des informations de signes vitaux du sujet, dans lequel les capteurs de signes vitaux sont portés par le corps de mise en prise ; et
la détermination de niveaux de couplage pour des capteurs individuels des capteurs de signes vitaux sur la base des signaux de couplage ;
la détermination d'états de signes vitaux multiples du sujet sur la base des signaux de sortie et des niveaux de couplage déterminés ; et
la détermination d'un indice de fiabilité de couplage sur la base des niveaux de couplage pour les capteurs individuels des capteurs de signes vitaux.

2. Procédé selon la revendication 1, dans lequel l'indice de fiabilité de couplage indique la fiabilité des états de signes vitaux multiples déterminés du sujet.

3. Procédé selon la revendication 1, dans lequel les capteurs de signes vitaux incluent un capteur de température à flux de chaleur nul dans lequel la production de signaux de sortie transportant des informations de signes vitaux du sujet inclut :
la création, par un capteur de température à flux de chaleur nul, d'une isolation thermique entre le corps de mise en prise et le sujet ; et
la production, par le capteur de température à flux de chaleur nul, de signaux de sortie transportant une seconde température du sujet,
dans lequel la détermination d'états de signes vitaux multiples du sujet inclut la détermination d'une température fondamentale sur la base des signaux de sortie produits par le capteur de température à flux de chaleur nul.

4. Procédé selon la revendication 1, dans lequel les capteurs de couplage incluent un ensemble d'une ou plusieurs électrodes dans lequel la détermination d'un indice de fiabilité de couplage comprend :
le positionnement de l'ensemble d'une ou plusieurs électrodes adjacent au sujet ;
la formation d'une capacité, par l'ensemble d'une ou plusieurs électrodes, entre les électrodes et le sujet ; et
la détermination de l'indice de fiabilité de couplage sur la base de la capacité formée entre les électrodes et le sujet.

5. Procédé selon la revendication 1, dans lequel les capteurs de couplage incluent un premier ensemble d'une ou plusieurs bobines d'excitation et un second ensemble d'une ou plusieurs bobines d'excitation, dans lequel la détermination d'un indice de fiabilité de couplage comprend :
la production, par le premier ensemble d'une ou plusieurs bobines d'excitation, d'un premier ensemble d'un ou plusieurs champs magnétiques ;
le fait d'amener le sujet à produire un second ensemble d'un ou plusieurs champs magnétiques en réponse à une interaction avec le premier ensemble d'un ou plusieurs champs magnétiques ;
la détection, par un second ensemble d'une ou plusieurs bobines d'excitation, du second ensemble d'un ou plusieurs champs magnétiques produits par le sujet ;
la détermination de l'indice de fiabilité de couplage sur la base d'une différence entre le premier ensemble d'un ou plusieurs champs magnétiques et du second ensemble d'un ou plusieurs champs magnétiques.

6. Procédé selon la revendication 1, comprenant en outre l'étape de :
suivi de changements de l'indice de fiabilité de couplage de capteurs de signes vitaux individuels au cours du temps.

7. Système (10) pour la détermination et la régulation non invasives d'un ou plusieurs états de signes vitaux d'un sujet, le système comprenant :
des moyens (14) pour mettre en prise un sujet avec un corps ;
des moyens (16) pour produire des signaux de couplage transportant des informations de couplage avec le sujet, dans lesquels les moyens pour produire des signaux de couplage sont portés par les moyens pour mettre en prise le sujet avec le corps ;
des moyens (18) pour produire des signaux de sortie transportant des informations de signaux vitaux du sujet, dans lesquels les moyens pour produire des signaux de sortie sont portés par les moyens pour mettre en prise le sujet avec le corps ;
des moyens (111) pour déterminer des niveaux de couplage pour des moyens individuels des moyens pour produire des signaux de sortie transportant des informations de signes vitaux du sujet, sur la base des signaux de couplage produits par les moyens pour produire des signaux de couplage ;
des moyens (112) pour déterminer des états de signes vitaux multiples (46) du sujet sur la base des signaux de sortie provenant des moyens pour produire des signaux de sortie transportant des informations de signes vitaux du sujet et des niveaux de couplage déterminés ; et
des moyens (113) pour déterminer un indice de fiabilité de couplage sur la base des niveaux de couplage pour les moyens pour produire des signaux de sortie transportant des informations de signes vitaux du sujet.

8. Système selon la revendication 7, dans lequel l'indice (48) de fiabilité de couplage indique la fiabilité des états de signes vitaux multiples déterminés du sujet tels que déterminés par les moyens (112) pour déterminer des états de signes vitaux multiples (46) du sujet.

9. Système selon la revendication 7, dans lequel les moyens pour déterminer des états de signes vitaux multiples du sujet incluent :
des moyens (68) pour créer une isolation thermique entre le corps de mise en prise et le sujet, et
des moyens (20) pour produire des signaux de sortie transportant une première température du sujet,
dans lequel les moyens pour déterminer des états de signes vitaux multiples du sujet sont configurés pour déterminer une température fondamentale sur la base de la seconde température.

10. Système selon la revendication 7, dans lequel les moyens pour produire des signaux de couplage comprennent un moyen (22) pour former une capacité entre le moyen pour former une capacité et le sujet, et dans lequel les moyens pour déterminer l'indice de fiabilité de couplage sont basés sur la capacité formée entre les moyens pour former une capacité et le sujet.

11. Système selon la revendication 7, dans lequel les moyens pour produire des signaux de couplage transportant des informations de couplage comprennent :
un moyen (24) pour produire un premier ensemble d'un ou plusieurs champs magnétiques, dans lequel le premier ensemble d'un ou plusieurs champs magnétiques amène le sujet à produire un second ensemble d'un ou plusieurs champs magnétiques en réponse à une interaction par le premier ensemble d'un ou plusieurs champs magnétiques avec le sujet ; et
un moyen (28) pour détecter le second ensemble d'un ou plusieurs champs magnétiques produits par le sujet ;
dans lequel le moyen pour déterminer l'indice de fiabilité de couplage sur la base de la capacité formée entre une différence entre le premier ensemble d'un ou plusieurs champs magnétiques et le second ensemble d'un ou plusieurs champs magnétiques.

12. Système selon la revendication 11, dans lequel le moyen (24) pour produire un premier ensemble d'un ou plusieurs champs magnétiques comprend un premier ensemble d'une ou plusieurs bobines d'excitation et dans lequel le moyen (28) pour détecter le second ensemble d'un ou plusieurs champs magnétiques produits par le sujet comprend un second ensemble d'une ou plusieurs bobines d'excitation.

13. Système selon la revendication 7, comprenant en outre :
un moyen (114) pour suivre des changements de l'indice de fiabilité de couplage des moyens pour produire des signaux de sortie transportant des informations de signes vitaux d'un sujet au cours du temps.

14. Système selon la revendication 7, dans lequel les moyens pour produire des signaux de couplage sont portés par les moyens (14) pour mettre en prise le sujet avec le corps et comprennent des moyens pour transporter des informations se rapportant à un couplage électrique, thermique et/ou magnétique avec le sujet.
